(19) Europäisches Patentamt
European Patent Office
Office européen des brevets

(11) Veröffentlichungsnummer: **0 282 795 B1**

(12) **EUROPÄISCHE PATENTSCHRIFT**

(45) Veröffentlichungstag der Patentschrift: **17.04.91 Patentblatt 91/16**

(51) Int. Cl.[5]: **C07C 49/597,** C07C 49/647, C07C 45/74, C07C 45/67, C07C 45/84, C11B 9/00, A61K 7/46

(21) Anmeldenummer: **88102954.0**

(22) Anmeldetag: **27.02.88**

(54) **2-Alkyliden-3,3,5(3,5,5)-trimethylcyclopentanone als Riechstoffe.**

(30) Priorität: **06.03.87 DE 3707209**

(43) Veröffentlichungstag der Anmeldung: **21.09.88 Patentblatt 88/38**

(45) Bekanntmachung des Hinweises auf die Patenterteilung: **17.04.91 Patentblatt 91/16**

(84) Benannte Vertragsstaaten: **CH DE FR GB LI NL**

(56) Entgegenhaltungen:
**EP-A- 0 033 604**
**FR-A- 1 603 489**
**US-A- 2 069 861**

(73) Patentinhaber: **Henkel Kommanditgesellschaft auf Aktien
Postfach 1100 Henkelstrasse 67
W-4000 Düsseldorf-Holthausen (DE)**

(72) Erfinder: **Markert, Thomas, Dr.
Kölner Landstrasse 198
W-4000 Düsseldorf 13 (DE)**
Erfinder: **Bruns, Klaus, Prof. Dr.
Notburgaweg 6
W-4150 Krefeld-Traar (DE)**
Erfinder: **Krause, Horst-Jürgen, Dr.
Am Nettchesfeld 22
W-4000 Düsseldorf 13 (DE)**
Erfinder: **Penninger, Josef, Dr.
Mozartstrasse 64
W-4010 Hilden (DE)**
Erfinder: **Virnig, Michael, Dr.
1105 Lanewood Way
Santa Rosa CA 95404 (US)**
Erfinder: **Falk, Volker, Dr.
Krahnheide 64
W-5620 Velbert 15 (DE)**

EP 0 282 795 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft 2-Alkyliden-3,3,5(3,5,5,)-trimethylcyclopentanone, Verfahren zur deren Herstellung sowie die Verwendung derselben als Riechstoffe.

Jasmon, ein wesentlicher Träger des Jasmingeruches, ist in etherischen Ölen von Jasminum grandiflorum in Mengen von etwa 3% enthalten und soll auch in sehr geringen Mengen in Orangenblüten- und Jonquillenextraktölen vorkommen (L. Ruzicka, M. Pfeiffer in "Helvetica Chimica Acta" 16, 1208 (1933)). Zur Herstellung größerer Mengen dieses interessanten Riechstoffes werden jedoch zum einen sehr große Mengen an Jasminblüten benötigt, zum anderen erfordert die Isolierung von Jasmon langwierige und aufwendige Verfahrensschritte.

Die Aufgabe der Erfindung bestand daher in der Bereitstellung synthetischer Ersatzriechstoffe für Jasmon, die in technisch einfacher Weise in großen Mengen herstellbar sind.

Aus Bulletin de la Société Chimique de France 1959, 493 bis 6 ist 2-Benzyl-3,5,5-trimethylcyclopenten-2-on-1 bekannt, das bei der Umsetzung von 3,5,5-Trimethylcyclopenten-2-on-1 mit Benzylbromid in Gegenwart von Natrium-tert.-amylat entsteht. Es werden keine Geruchseigenschaften dieser Verbindung angegeben.

Die Erfindung geht von der überraschenden Feststellung aus, daß 2-Alkyliden-3,3,5(3,5,5)-trimethylcyclopentanone, die in 2-Stellung einen gesättigten oder ungesättigten, gerad- oder verzweigtkettigen Alkylrest mit 2 bis 7 C-Atomen besitzen, geeignete Ersatzriechstoffe für Jasmon sind. Sie können technisch einfach und in großen Mengen hergestellt werden.

Im folgenden werden unter dem Begriff "2-Alkyliden-3,3,5(3,5,5)-trimethylcyclopentanon" Isomerengemische verstanden, die sich aus 2-Alkyliden-3,3,5-trimethylcyclopentanonen oder 2-Alkyliden-3,5,5-trimethylcyclopentanon und 2-Alkyl-3,5,5-trimethylcyclopent-2-en-1-onen oder 2-Alkyliden-3,3,5-trimethylcyclopentanonen, 2-Alkyliden-3,5,5-trimethylcyclopentanonen und 2-Alkyl-3,5,5-trimethylcyclopenten-2-en-1-onen zusammensetzen.

Gegenstand der Erfindung sind 2-Alkyliden-3,3,5(3,5,5)-trimethylcyclopentanone der allgemeinen Formel

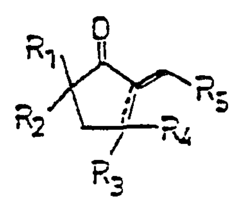

in der 3 der Reste $R_1$ bis $R_4$ $CH_3$, einer der Reste $R_1$ bis $R_4$ H und $R_5$ ein gesättigter oder ungesättigter, gerad- oder verzweigtkettiger Alkylrest mit 1 bis 7 C-Atomen bedeuten und sich die Doppelbindung in endo- oder exo-Stellung zum Cyclopentanongerüst befindet.

Ein weiterer Erfindungsgegenstand ist ein Verfahren zum Herstellung von 2-Alkyliden-3,3,5(3,5,5)-trimethylcyclopentanonen der allgemeinen Formel

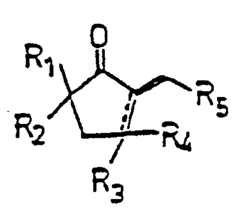

in der 3 der Reste $R_1$ bis $R_4$ $CH_3$, einer der Reste $R_1$ bis $R_4$ H und $R_5$ ein gesättigter oder ungesättigter, gerad- oder verzweigtkettiger Alkylrest mit 1 bis 7 C-Atomen bedeuten und sich die Doppelbindung in endo- oder exo-Stellung zum Cyclopentanongerüst befindet, das dadurch gekennzeichnet ist, daß man in einer Aldolkondensation 2,2,4-Trimethylcyclopentanon, 2,4,4-Trimethylcyclopentanon oder deren Gemische mit überschüssigem Aldehyd $R_5$-CHO in Gegenwart von Basen zur Reaktion bringt, anschließend neutralisiert und gewünschtenfalls das erhaltene Reaktionsprodukt unter Zusatz von Halogenwasserstoff, vorzugsweise Bromwasserstoff isomerisiert.

Gegenstand der Erfindung ist ein weiteres Verfahren zur Herstellung von 2-Alkyliden-3,3,5(3,5,5)-trimethylcyclopentanonen der allgemeinen Formel

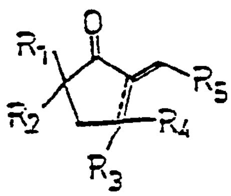

in der drei der Reste $R_1$-$R_4$ $CH_3$, einer der Reste $R_1$-$R_4$ H und $R_5$ ein gesättigter oder ungesättigter gerad- oder verzweigtkettiger Alkylrest mit 1 bis 7 C-Atomen bedeuten und sich die Doppelbindung in endo- oder exo-Stellung zum Cyclopentanongerüst befindet, das dadurch gekennzeichnet ist, daß man in einer Aldolkondensation 2,2,4-Trimethylcyclopentanon, 2,4,4-Trimethylcyclopentanon oder deren Gemische mit überschüssigem Aldehyd $R_5$-CHO in Gegenwart von Basen sowie Phasentransferkatalysatoren zur Reaktion bringt, das Reaktionsgemisch mit einem organischen Lösungsmittel extrahiert, anschließend neutralisiert und trocknet, den Rückstand mit einem mit Wasser ein Azeotrop bildendem Lösungsmittel und sauren Katalysatoren, vorzugsweise p-Toluolsulfonsäure und/oder Amidosulfonsäure behandelt und gewünschtenfalls das erhaltene Reaktionsprodukt unter Zusatz von Halogenwasserstoff, vorzugsweise Bromwasserstoff isomerisiert.

Erfindungsgegenstand ist ferner die Verwendung von 2-Alkyliden-3,3,5,(3,5,5)-trimethylcyclopentanonen der allgemeinen Formel

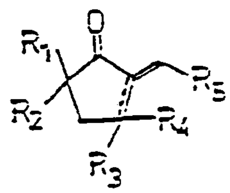

in der 3 der Reste $R_1$ bis $R_4$ $CH_3$, einer der Reste $R_1$ bis $R_4$ H und $R_5$ ein gesättigter oder ungesättigter, gerad- oder verzweigtkettiger Alkylrest mit 1 bis 7 C-Atomen bedeuten und sich die Doppelbindung in endo- oder exo-Stellung zum Cyclopentanongerüst befindet, als Riechstoffe.

Vorzugsweise werden 2-Alkyliden-3,3,5(3,5,5)-trimethylcyclopentanone mit 2 bis 5 C-Atomen im gesättigten oder ungesättigten, gerad- oder verzweigtkettigen $R_5$-Rest eingesetzt. Besonders bevorzugt werden 2-Alkyliden-3,3,5(3,5,5)-trimethylcyclopentanone mit 3 C-Atomen im gesättigten oder ungesättigten, gerad- oder verzweigtkettigen $R_5$-Rest, beispielsweise 2-n-Butyliden-3,3,5(3,5,5)-trimethylcyclopentanon.

Die Herstellung der erfindungsgemäßen Cyclopentanone erfolgt in an sich bekannter Weise durch Aldolkondensation, indem 2,2,4-Trimethylcyclopentanon, 2,4,4-Trimethylcyclopentanon oder deren Gemische mit überschüssigem Aldehyd $R_5$-CHO mit $R_5$ = gesättigter oder ungesättigter, gerad- oder verzweigtkettiger Alkylrest mit 1 bis 7 C-Atomen in Gegenwart von Basen, zur Reaktion gebracht, vorzugsweise anschließend 10-20 Stunden, insbesondere 14-17 Stunden, bei 20-25°C gerührt werden. Als Basen werden vorzugsweise Alkalihydroxide, -methylate, -ethylate, -propylate und/oder -butylate, besonders bevorzugt Natriummethylat, eingesetzt. Die Alkolkondensation zur Herstellung der erfindungsgemäßen Cyclopentanone kann auch in folgender Weise durchgeführt werden : 2,2,4-Trimethylcyclopentanon, 2,4,4-Trimethylcyclopentanon oder deren Gemische werden mit überschüssigem Aldehyd $R_5$-CHO in Gegenwart von Basen sowie Phasentransferkatalysatoren zur Reaktion gebracht, vorzugsweise 10 bis 20 Stunden, insbesondere 14 bis 17 Stunden bei 20 bis 25°C stehen gelassen, das Reaktionsgemisch mit organischen Lösungsmitteln, beispielsweise Diethylether extrahiert, anschließend mit beispielsweise Natriumchlorid neutralisiert und mit beispielsweise Natrium-, Calcium- und/oder Magnesiumsulfat getrocknet und der Rückstand mit einem mit Wasser ein Azeotrop bildendem Lösungsmittel, beispielsweise Toluol, und sauren Katalysatoren, vorzugsweise p-Toluolsulfonsäure und/oder Amidosulfonsäure, bei Siedetemperatur des Lösungsmittels behandelt. Als Basen werden vorzugsweise Alkalihydroxide, besonders bevorzugt Kaliumhydroxid, eingesetzt. Kronenether und/oder Polyalkylenglycole sind bevorzugte Phasentransferkatalysatoren ; besonders bevorzugt werden Polyethylenglycole mit mittleren Molekulargewichten von 400 bis 1 000. Die Aldolkondensationen werden vorzugsweise bei 20 bis 30°C durchgeführt. Die neutralisierten und von Lösungsmitteln befreiten Reaktionsprodukte enthalten unter anderem 2-Alkyliden-3,5,5-trimethylcyclopentanon, d.h. ein Trimethylcyclopentanonderivat mit exo-cyclischer Doppelbindung. Um Reaktionsprodukte mit einem höheren Gehalt an Trimethylcyclopentanonderivaten mit endo-cyclischer Doppelbindung, d.h. mit einem höheren Gehalt an 2-Alkyl-3,5,5-trimethylcyclopent-2-en-1-on zu erhalten, kann eine Isomerisierung in Gegenwart von Halogenwasserstoffen, beispielsweise Bromwasserstoff

bei 80 bis 85°C nach bekannten Methoden durchgeführt werden (L.-F. Tietze, Th. Eicher in "Reaktionen und Synthesen", Seite 162, Thieme-Verlag Stuttgart 1981).

Die erfindungsgemäßen 2-Alkyliden-3,3,5(3,5,5)-trimethylcyclopentanone sind ausgezeichnete Jasmonriechstoffe mit einem sehr harmonischen Geruchsbild. Sie können alleine oder in Kombination mit anderen Riechstoffen zur Parfümierung von beispielsweise Kosmetika, wie Duftwässern, Cremes, Lotionen, Aerosolen und Toilettenseifen, in der Extraitparfümerie, zur Geruchsverbesserung technischer Artikel, wie Reinigungs- und Desinfektionsmitteln, sowie in Textilbehandlungsmitteln verwendet werden.

In Riechstoffkompositonen liegt der Gehalt an den erfindungsgemäßen Riechstoffen zwischen 1 und 50 Gew.-%, vorzugsweise zwischen 2 und 25 Gew.-%.

Beispiele

Beispiel 1

2-n-Butyliden-3,5,5(3,5,5)-trimethylcyclopentanon

Ansatz:

| | | |
|---|---|---|
| I | 170,37 g (1,35 Mol) | 2,2,4(2,4,4)-Trimethylcyclo pentanon (Isomerenverhältnis = 57,5 : 42,5) |
| II | 72 g (0,4 Mol) | Natriummethylat 30 %ig |
| III | 71 g (0,5 Mol) | Natriumsulfat wasserfrei |
| IV | 194,7 g (2,7 Mol) | n-Butanal |

Ausführung :

Zu einer Mischung von I, II und III wird unter Stickstoff und Rühren IV in 6 Stunden kontinuierlich zugetropft. Man rührt weiter über Nacht und neutralisiert anschließend mit verdünnter $H_2SO_4$. Die Reaktionsmischung, die ausgefallene Salze enthält, wird mit Wasser verdünnt, die wäßrige Phase abgetrennt und die organische Phase eingeengt. Der Rückstand wird destilliert.

Ausbeute :
124,3 g, 51,1% der Theorie (d.Th.)
Siedepunkt :
55-58°C / 0,9 mbar
Geruch :
Jasmon, Sellerie, Maggi

Beispiel 2

2-n-Butyliden-3,3,5(3,5,5)-trimethylcyclopentanon

Ansatz:

| | | |
|---|---|---|
| I | 126,2 g (1 Mol) | 2,2,4(2,4,4)-Trimethylcyclopentanon (Isomerenverhältnis = 57,5 : 42,5) |
| II | 26,4 g (0,4 Mol) | Kaliumhydroxid 85 %ig |
| III | 5,2 g | Polyethylenglykol, mittleres Molekulargewicht = 600 |
| IV | 44,17 g (0,35 Mol) | 2,2,4(2,4,4)-Trimethylcyclopentanon (Isomerenverhältnis = 57,5 : 42,5) |
| V | 97,3 g (1,35 Mol) | n-Butanal |
| VI | 0,2 g | p-Toluolsulfonsäuremonohydrat |

Ausführung :

I, II und III werden unter Rühren und Stickstoffatmosphäre in 6 Stunden kontinuierlich mit einer Mischung von IV und V versetzt. Man läßt über Nacht stehen, neutralisiert nicht, sondern extrahiert mit Ether, wäscht mit gesättigter Kochsalzlösung neutral und engt nach dem Trocknen über $Na_2SO_4$ ein. Der Rückstand wird in Toluol aufgenommen und mit 0,2 g VI am Wasserabscheider zum Rückfluß erhitzt.

Es werden 7,8 ml Wasser azeotrop abdestilliert. Nach Auswaschen von VI wird das Toluol abdestilliert und der Rückstand fraktioniert.

Ausbeute :

71,9 g, 29,5% d.Th.

Beispiel 3

2-n-Butyl-3,5,5-trimethylcyclopent-2-en-1-on (als Isomerengemisch mit 2-n-Butyliden-3,3,5-Trimethycyclopentanon) aus 2-n-Butyliden-3,3,5(3,5,5)-trimethylcyclopentanon durch Isomerisierung

Ansatz:

| | | |
|---|---|---|
| I | 50 g | 2-n-Butyliden-3,3,5(3,5,5)-trimethylcyclopentanon, hergestellt nach Beispiel 1 |
| II | 300 ml | n-Butanol |
| III | 50 ml | Bromwasserstoffsäure 48 %ig |

Ausführung :

I und II werden gemischt und unter Rühren und Stickstoffatmosphäre auf 80°C erhitzt. Anschließend wird III langsam zugetropft und danach noch 14 Stunden unter Rückfluß erhitzt. Nach dem Abkühlen wird mit Ether extrahiert, neutral gewaschen, getrocknet, eingeengt und destilliert. Man erhält 48 g Isomerengemisch : das Verhältnis endo- zu exocyclischer Verbindung beträgt 28,4 : 51,6%.

Siedepunkt :
52-53°C / 1 mbar
Geruch :
Jasmon-, Cord-Note

Beispiel 4

2-n-Pentyliden-3,5,5(3,3,5)-trimethyl-cyclopentanon

Ansatz:

| | | |
|---|---|---|
| I | 94,7 g (0,75 Mol) | 2,2,4(2,4,4)-Trimethylcyclopentanon (Isomerenverhältnis = 57,5 : 42,5) |
| II | 54 g (0,3 Mol) | Natriummethylat 30 %ig |
| III | 53,3 g (0,375 Mol) | Natriumsulfat wasserfrei |
| IV | 31,6 g (0,25 Mol) | 2,2,4(2,4,4)-Trimethylcyclopentanon (Isomerenverhältnis = 57,5 : 42,5) |
| V | 172,2 g (2 Mol) | n-Pentanal |

Ausführung :

I, II und III werden bei 22°C unter Stickstoff gerührt und eine Mischung aus IV und V wird in 6 Stunden zugetropft. Man rührt über Nacht und neutralisiert mit verdünnter Schwefelsäure. Durch Zusatz von Wasser werden ausgefallene Salze gelöst, die wäßrige Phase wird abgetrennt und die organische Phase eingeengt. Danach wird destilliert, anschließend fraktioniert.
Ausbeute :
95 g, 48,9% d. Th.
Siedepunkt der Pentylidenverbindung :
60-70% / 1 mbar
Geruch :
Jasmon-Note, fruchtig

Beispiel 5

2-n-Butyliden-3,5,5-trimethylcyclopentanon

Ansatz:

| | | |
|---|---|---|
| I | 40 g (0,318 Mol) | 2,2,4-Trimethylcyclopentanon 100%ig |
| II | 17,1 g (0,095 Mol) | Natriummethylat 30%ig |
| III | 17 g (0,12 Mol) | Natriumsulfat wasserfrei |
| IV | 46,2 g (0,64 Mol) | n-Butanol |

Ausführung analog Beispiel 1
Ausbeute :
42,3 g, 73,3% d. Th.
Siedepunkt :
60-63°C / 1 mbar
Geruch :

feine Jasmon-, Cord-Note

Beispiel 6

2n-Butyliden-3,3,5-trimethylcyclopentanon

Ansatz:

| | | |
|---|---|---|
| I | 40,0 g (0,318 Mol) | 2,4,4-Trimethylcyclopentanon 99,9%ig |
| II | 18,1 g (0,095 Mol) | Natriummethylat 30%ig |
| III | 17 g (0,12 Mol) | Natriumsulfat wasserfrei |
| IV | 46,2 g (0,64 Mol) | n-Butanol |

Ausführung analog Beispiel 1
Ausbeute :
27,2 g, 47% d. Th.
Siedepunkt :
63-65°C / 1 mbar
Geruch :
strahlende Sellerie-, Maggi-Note, würzig, grün

## Kompositionsbeispiele

### Jasmin-Akkord

Kompositionsbeispiele

Jasmin-Akkord

| | |
|---|---|
| Methylcyclooctylcarbonat (Jasmacyclat$^{R}$, Henkel KGaA) | 330 Gew.-Teile |
| alpha-Hexylzimtaldehyd | 200 Gew.-Teile |
| Linalool | 60 Gew.-Teile |
| Linalylacetat | 60 Gew.-Teile |
| Hydroxycitronellal | 60 Gew.-Teile |
| Benzylacetat | 50 Gew.-Teile |
| 2-n-Butyliden-3,5,5(3,5,5)-trimethylcyclopentanon, hergestellt nach Beispiel 1 | 50 Gew.-Teile |
| Benzylsalicylat | 50 Gew.-Teile |
| Ylangöl | 40 Gew.-Teile |
| Geranylacetat | 25 Gew.-Teile |
| alpha-Methyl-ß(p-tert.-butylphenyl)propionaldehyd | 20 Gew.-Teile |
| Phenylethylacetat | 20 Gew.-Teile |
| Methyljonongemisch (Isoraldein 70) | 15 Gew.-Teile |
| p-Kresylphenylacetat, 10 % in Diethylphthalat | 15 Gew.-Teile |
| 6-(Spiro-endo-methylen 1,4-cyclohexyl-2)tetrahydropyran (Mugoflor, H & R) | 5 Gew.-Teile |

<u>Rose-Jasmin-Akkord</u>

| | |
|---|---|
| Citronellol | 230 Gew.-Teile |
| Hexylsalicylat | 220 Gew.-Teile |
| 2-n-Pentyliden-3,3,5(3,5,5)-trimethylcyclopentanon | 100 Gew.-Teile |
| Phenylethylalkohol | 90 Gew.-Teile |
| Methyljonongemisch (Isoraldein 70) | 75 Gew.-Teile |
| alpha-Hexylzimtaldehyd | 70 Gew.-Teile |
| Ylangöl | 60 Gew.-Teile |
| n-Iso-undecanol | 50 Gew.-Teile |
| Geraniumöl Bourbon | 40 Gew.-Teile |
| Benzylacetat | 40 Gew.-Teile |
| Indol 10 % in Benzylalkohol | 15 Gew.-Teile |
| Geranonitril | 10 Gew.-Teile |

## Ansprüche

1. 2-Alkyliden-3,3,5(3,5,5)-trimethylcyclopentanone der allgemeinen Formel

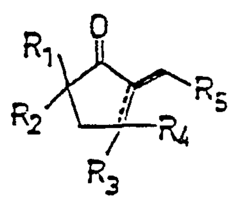

in der drei der Rest $R_1$ bis $R_4$ $CH_3$, einer der Reste $R_1$ bis $R_4$ H und $R_5$ ein gesättigter oder ungesättigter, gerad- oder verzweigtkettiger Alkylrest mit 1 bis 7 C-Atomen bedeuten und sich die Doppelbindung in endo- oder exo-Stellung zum Cyclopentanongerüst befindet.

2. 2-Alkyliden-3,3,5(3,5,5)-trimethylcyclopentanone nach Anspruch 1, dadurch gekennzeichnet, daß $R_5$ ein gesättigter oder ungesättigter, gerad- oder verzweigtkettiger Alkylrest mit 2 bis 5 C-Atomen ist.

3. 2-Alkyliden-3,3,5(3,5,5)-trimethylcyclopentanone nach einem oder beiden der Ansprüche 1 und 2, dadurch gekennzeichnet, daß $R_5$ ein gesättigter oder ungesättigter, gerad- oder verzweigtkettiger Alkylrest mit 3 C-Atomen ist.

4. Verfahren zur Herstellung von 2-Alkyliden-3,3,5(3,5,5)-trimethylcyclopentanonen nach einem der mehreren der Ansprüche 1 bis 3, dadurch gekennzeichnet, daß man in einer Aldolkondensation 2,2,4-Trimethylcyclopentanon, 2,4,4-Trimethylcyclopentanon, oder deren Gemische mit überschüssigem Aldehyd $R_5$-CHO in Gegenwart von Basen zur Reaktion bringt, anschließend neutralisiert und gewünschtenfalls das erhaltene Reaktionsprodukt unter Zusatz von Halogenwasserstoffen, vorzugsweise Bromwasserstoff, isomerisiert.

5. Verfahren nach Anspruch 4, dadurch gekennzeichnet, daß man als Basen Alkalihydroxide, -methylate, -ethylate, -propylate und/oder -butylate, vorzugsweise Natriummethylat, einsetzt.

6. Verfahren zur Hersellung von 2-Alkyliden-3,3,5(3,5,5)-trimethylcyclopentanonen nach einem oder mehreren der Ansprüche 1 bis 3, dadurch gekennzeichnet, daß man in einer Aldolkondensation 2,2,4-Trimethylcyclopentanon, 2,4,4-Trimethylcyclopentanon oder deren Gemische mit überschüssigem Aldehyd $R_5$-CHO in Gegenwart von basen sowie Phasentransferkatalysatoren zur Reaktion bringt, das Reaktionsgemisch mit organischen Lösungsmitteln extrahiert, anschließend neutralisiert und trocknet, den Rückstand mit einem mit Wasser ein Azeotrop bildendem Lösungsmittel und sauren Katalysatoren, vorzugsweise p-Toluolsulfonsäure und/oder Amidosulfonsäure, behandelt und gewünschtenfalls das erhaltene Reaktionsprodukt unter Zusatz

von Halogenwasserstoffen, vorzugsweise Bromwasserstoff, isomerisiert.

7. Verfahren nach Anspruch 6, dadurch gekennzeichnet, daß man als Basen Alkalihydroxyde, vorzugsweise Kaliumhydroxyd, und als Phasentransferkatalysatoren Kronenether und/oder Polyalkylenglycole, vorzugsweise Polyethylenglycole mit mittleren Molekulargewichten von 400 bis 1000 einsetzt.

8. Verwendung von 2-Alkyliden-3,3,5(3,5,5)-trimethylcyclopentanonen nach einem oder mehreren der Ansprüche 1 bis 3 als Riechstoffe.

## Claims

1. 2-Alkylidene-3,3,5(3,5,5)-trimethyl cyclopentanones corresponding to the following general formula

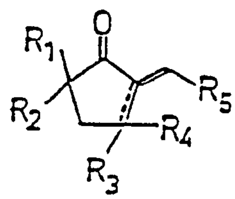

in which three of the substituents $R_1$ to $R_4$ are $CH_3$, one of the substituents $R_1$ to $R_4$ is H and $R_5$ is a saturated or unsaturated, linear or branched $C_1$-$C_7$ alkyl radical ; and the double bond is in the endo or exo position to the cyclopentanone structure.

2. 2-Alkylidene-3,3,5(3,5,5)-trimethyl cyclopentanones as claimed in claim 1, characterized in that $R_5$ is a saturated or unsaturated, linear or branched $C_2$-$C_5$ alkyl radical.

3. 2-Alkylidene-3,3,5(3,5,5)-trimethyl cyclopentanones as claimed in one or both of claims 1 and 2, characterized in that $R_5$ is a saturated or unsaturated, linear or branched $C_3$ alkyl radical.

4. A process for the preparation of the 2-alkylidene-3,3,5(3,5,5)-trimethyl cyclopentanones claimed in one or more of claims 1 to 3, characterized in that, in an aldol condensation, 2,2,4-trimethyl cyclopentanone, 2,4,4-trimethyl cyclopentanone or mixtures thereof are reacted with excess aldehyde $R_5$-CHO in the presence of bases, the reaction product obtained is subsequently neutralized and, if desired, isomerized in the presence of hydrogen halides, preferably hydrogen bromide.

5. A process as claimed in claim 4, characterized in that alkali hydroxides, methylates, ethylates, propylates and/or butylates, preferably sodium methylate, are used as the bases.

6. A process for the preparation of the 2-alkylidene-3,3,5(3,5,5)-trimethyl cyclopentanones claimed in one or more of claims 1 to 3, characterized in that in an aldol condensation, 2,2,4-trimethyl cyclopentanone, 2,4,4-trimethyl cyclopentanone or mixtures thereof are reacted with excess aldehyde $R_5$-CHO in the presence of bases and phase transfer catalysts, the reaction mixture is extracted with organic solvents, subsequently neutralized and dried, the residue is treated with a solvent which forms and azeotrope with water an acidic catalysts, preferably p-toluenesulfonic acid and/or amidosulfonic acid, and, if desired, the reaction product obtained is isomerized in the presence of hydrogen halides, preferably hydrogen bromide.

7. A process as claimed in claim 6, characterized in that alkali hydroxides, preferably potassium hydroxide, are used as the bases and crown ethers and/or polyalkylene glycols, preferably polyethylene glycols having average molecular weights of 400 to 1,000, are used as the phase transfer catalysts.

8. The use of the 2-alkylidene-3,3,5(3,5,5)-trimethyl cyclopentanones as claimed in one or more of claims 1 to 3 as perfumes.

## Revendications

1. 2-alcoylidène-3,3,5(3,5,5)-triméthylcyclopentanones de formule générale :

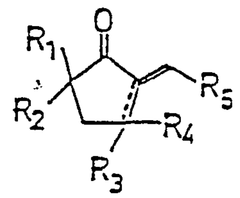

dans laquelle trois des radicaux $R_1$ à $R_4$ représentent un méthyle, un des radicaux $R_1$ à $R_4$ représente de l'hydrogène, et $R_5$ représente un radical alcoyle saturé ou non saturé, à chaîne linéaire ou ramifiée, ayant de 1 à 7 atomes de carbone et la double liaison se trouve en position endo ou exo par rapport au squelette de la cyclopentanone.

2. 2-alcoylidène-3,3,5-(3,5,5)-triméthylcyclopentanone selon la revendication 1, caractérisée en ce que $R_5$ est un radical alcoyle saturé ou non saturé, à chaîne linéaire ou ramifiée ayant de 2 à 5 atomes de carbone.

3. 2-Alcoylidène-3,3,5-(3,5,5)-triméthylcyclopentanone selon l'une ou les deux revendications 1 et 2, caractérisée en ce que $R_5$ est un radical alcoyle saturé ou non saturé à chaîne linéaire ou ramifiée ayant 3 atomes de Carbone.

4. Procédé d'obtention de 2-alcoylidène-3,3,5-(3,5,5)-triméthylcyclopentanones selon l'une ou plusieurs des revendications 1 à 3, caractérisé en ce que l'on met en réaction dans une condensation aldolique la 2,2,4-triméthylcyclopentanone, la 2,4,4-triméthylcyclopentanone ou leurs mélanges avec un aldéhyde $R\text{-}_5CHO$ en excès en présence de bases, ensuite neutralise et si désiré isomérise le produit de la réaction par addition d'acides halohydriques, de préférence de l'acide bromhydrique.

5. Procédé selon la revendication 4, caractérisé en ce que l'on met en oeuvre comme bases, des hydroxydes alcalins, des méthylates alcalins, des éthylates alcalins, des propylates alcalins, et/ou des butylates alcalins, de préférence le méthylate de sodium.

6. Procédé d'obtention des 2-alcoylidène-3,3,5-(3,5,5)-triméthylcyclopentanones selon l'une ou plusieurs revendications 1 à 3, caractérisé en ce que l'on met en réaction dans une condensation aldolique la 2,2,4-triméthylcyclopentanone, la 2,4,4-triméthylcyclopentanone ou leurs mélanges avec un aldéhyde $R\text{-}_5CHO$ en excès en présence de bases ainsi que de catalyseurs de transfert de phase, on extrait le mélange réactionnel avec des solvants organiques, que l'on neutralise ensuite et sèche, traite le résidu avec un solvant qui forme avec l'eau un mélange azéotropique et des catalyseurs acides, de préférence l'acide p-toluène sulfonique et/ou l'acide amidosulfonique, et que l'on isomère si désiré le produit de réaction obtenu par addition d'acides halohydriques, de préférence l'acide bromhydrique.

7. Procédé selon la revendication 6, caractérisé en ce que l'on met en oeuvre comme base des hydroxydes alcalins, de préférence l'hydroxyde de potassium et comme catalyseurs de transfert de phase un éther-couronne (Crown éther) et/ou un polyalcoylèneglycol, de préférence des polyéthylèneglycols ayant un poids moléculaire moyen de 400 à 1000.

8. Utilisation de 2-alcoylidene -3,3,5-(3,5,5)-triméthylcyclopentanones selon l'une ou plusieurs des revendications 1 à 3, comme parfums.